# EUROPÄISCHE PATENTSCHRIFT

(11) **EP 1 866 008 B1**
(45) Veröffentlichungstag und Bekanntmachung des Hinweises auf die Patenterteilung: **16.04.2014**
(21) Anmeldenummer: 06701652.7
(22) Anmeldetag: 06.02.2006
(51) Int. Cl.: A61M 1/06, A61M 39/24, F16K 15/14

(54) **VENTIL, INSBESONDERE FÜR EIN BRUSTHAUBENSET UND BRUSTHAUBENSET MIT VENTIL**
VALVE, ESPECIALLY FOR A BREAST CUP SET, AND BREAST CUP SET WITH VALVE
SOUPAPE, EN PARTICULIER POUR UN ENSEMBLE TIRE-LAIT, ET ENSEMBLE TIRE-LAIT AVEC SOUPAPE

(30) Priorität: 07.04.2005 CH 638052005; 13.06.2005 US 689842 P
(43) Veröffentlichungstag der Anmeldung: 19.12.2007
(73) Patentinhaber: Medela Holding AG, 6340 Baar (CH)
(72) Erfinder: VÖGELIN, Stefan, CH-5644 Auw (CH); STADELMANN, Urs, CH-5735 Pfeffikon (CH)
(74) Vertreter: Clerc, Natalia
(86) Internationale Anmeldenummer: PCT/CH2006/000070
(87) Internationale Veröffentlichungsnummer: WO 2006/105675

(56) Entgegenhaltungen:
- EP-A- 1 099 455
- WO-A-03/092768
- DE-A1- 19 747 842
- US-A- 5 025 829
- US-A1- 2004 250 864

## Beschreibung

### Technisches Gebiet

Die Erfindung betrifft ein Ventil gemäss Oberbegriff des Patentanspruchs 1. Das Ventil ist insbesondere zur Verwendung in einem Brusthaubenset geeignet. Die Erfindung betrifft ferner ein Brusthaubenset zum Abpumpen von menschlicher Muttermilch gemäss Oberbegriff des Patentanspruchs 16.

### Stand der Technik

Brusthaubensets werden zusammen mit Brustpumpen zum Abpumpen von menschlicher Muttermilch eingesetzt. Sie bestehen üblicherweise aus einer Brusthaube mit einem Brusthaubentrichter zur Auflage an die menschliche Mutterbrust, einem ersten Anschlussteil zur Verbindung mit einem Milchsammelbehälter und einem zweiten Anschlussteil zum Anschluss an eine mechanische oder elektrische Pumpe bzw. zum Anschluss an eine Saugleitung zu einer derartigen Pumpe.

Zum Abpumpen der Muttermilch wird der Brusthaubentrichter möglichst dicht auf die Mutterbrust aufgelegt und mittels der Pumpe zyklisch ein Vakuum im Brusthaubentrichter erzeugt. Um den zu evakuierenden Raum möglichst klein zu halten, d.h. das Totvolumen zu minimieren, weist das Brusthaubenset zum Milchsammelbehälter hin ein Rückschlagventil auf. Dieses wird durch den Druck der abgesaugten Milch zum Innenraum des Milchsammelbehälters hin geöffnet und verschliesst diesen bei angelegtem Vakuum gegenüber dem evakuierten Volumen des restlichen Brusthaubensets.

Das Ventil weist einen Ventilsitz auf, welcher von einem Ventilkörper in Form einer einseitig befestigten Membranklappe bedeckt ist. Bei genügendem Druck der einströmenden Milch wird die Membranklappe zum Behälterinnenraum hin geöffnet. Dieses Ventil hat sich zwar in der Praxis bewährt. Die Herstellung derartiger Brusthaubensets ist aber relativ teuer. Da der Markt vermehrt fordert, diese Brusthaubensets aus hygienischen Gründen nur einmal oder zumindest nur wenige Male zu benützen, ist es somit ein Bedürfnis, die Herstellungskosten möglichst zu senken.

Da diese Brusthaubensets somit maximal nur einige wenige Male benützt werden, sollten die einzelnen Teile davon möglichst kostengünstig herstellbar sein. Dies gilt insbesondere auch für das Rückschlagventil. Da jedoch das Brusthaubenset nach jedem Gebrauch gereinigt werden sollte, muss das Ventil zudem möglichst einfach aufgebaut und entsprechend leicht zu reinigen sein. Des weiteren darf es sich bei der Reinigung nicht verziehen, um auch anschliessend eine genügende Dichtung zu gewährleisten. Die Form, Ausgestaltung und Materialwahl des Ventils spielt bei der Erfüllung dieser Aufgaben eine wesentliche Rolle. Üblicherweise sind die Brusthaubensets aus Kunststoff, insbesondere Polyethylen (PE), Polypropylen (PP) oder Polycarbonat (PC) gefertigt, wobei der Ventilkörper selber aus Silikon besteht.

EP 1 099 455 offenbart ein Ventil für medizinische Infusionen mit einer Ventilmembran welche gleichmässig entlang eines Kreises verteilte Öffnungen aufweist.

### Darstellung der Erfindung

Es ist deshalb eine Aufgabe der Erfindung, ein Ventil zu schaffen, welches kostengünstig herstellbar ist und trotzdem einen sicheren Verschluss ermöglicht.

Diese Aufgabe löst ein Ventil mit den Merkmalen des Patentanspruchs 1.

Das erfindungsgemässe Ventil weist einen Ventilsitz und einen Ventilkörper mit einer kreisförmigen Membran auf. Der Ventilkörper ist über dem Ventilsitz angeordnet, um diesen bei Auflage auf dem Ventilsitz dichtend zu verschliessen. Der Ventilsitz und der Ventilkörper weisen Öffnungen auf, welche versetzt zueinander angeordnet sind und bei Anhebung der Membran des Ventilkörpers einen freien Durchgang bilden. Die Membran des Ventilkörpers weist langgezogene Öffnungen auf, welche gleichmässig verteilt entlang eines Kreises in der Peripherie der Membran angeordnet sind, wobei der Kreis vorzugsweise annähernd denselben Mittelpunkt aufweist wie die Membran. Die langgezogenen Öffnungen sind bogenformig gestaltet, wobei sich ihre Längsausdehnung entlang des erwähnten Kreises erstreckt. Sie sind durch Stege voneinander getrennt. Mit anderen Worten bilden die langgezogenen Öffnungen einen gemeinsamen Kreisring, dessen Breite ein Vielfaches kleiner als der kleinere Radius des Kreisrings ist und welcher mit Stegen versehen ist. Die Membran ist im Bereich benachbart zu diesen Stegen geschwächt ausgebildet, wobei benachbart zu den Stegen kompakte, T-förmige Öffnungen vorhanden sind.

Vorzugsweise sind genau drei derartige Öffnungen und genau drei Stege vorhanden, so dass die Membran in einer peripheren Dreipunktaufhängung gehalten ist.

Die geschwächten Bereiche können zusätzlich verdünnte Stellen sein. Diese geschwächten Bereiche dienen dazu, Spannungen auszugleichen, so dass die Funktionalität des Ventils auch nach einer hitzebeaufschlagten Reinigung noch gewährleistet ist.

Gute Resultate wurden mit T-förmigen kompakten Öffnungen erzielt.

Die Kombination von verdünnten Bereichen und kompakten Öffnungen hat den Vorteil, dass die das Gelenk des Ventils bildenden Stege auch nach der Reinigung genügend flexibel sind, um den durch die langgezogenen Öffnungen gebildeten Membrankreis genügend schnell und gezielt anzuheben und abzusenken

Vorzugsweise ist an der Membran ein zylinderförmiger Mantel angeformt, welcher über einen Kragen des Ventilsitzes gestülpt ist. Weist der Mantel auf seiner Innenseite axial verlaufende Kerben und/oder umlaufende Nuten auf wird ebenfalls ein Verzug bei der Reinigung vermieden.

Das beschriebene erfindungsgemässe Ventil in den oben genannten und auch nachfolgend beschriebenen Ausfühnmgsformen wird bevorzugt in Brusthaubensets der eingangs genannten Art verwendet. Es lässt sich jedoch auch in anderen Artikeln, insbesondere medizinischen Produkten, verwenden, wie beispielsweise für Drainagebeutel zur Absaugung von Körperflüssigkeiten oder in Absaugschläuchen jedweleher Art.

Es ist deshalb eine weitere Aufgabe der Erfindung, ein möglichst kostengünstig herstellbares Brusthaubenset zu schaffen.

Diese Aufgabe löst ein Brusthaubenset mit den Merkmalen des Patentanspruch 16.

Um die Hygienevorschriften für einen Mehrfachgebrauch zu erfüllen sind Bruthaubensets üblicherweise sterilisierbar und autoklavierbar. Erhöhte Anforderungen an die Hygiene sowohl im Spital- wie auch im Privatgebrauch haben jedoch in letzter Zeit das Bedürfnis nach Brusthaubensets geweckt, welche nur noch einen beschränkten Mehrfachgebrauch zulassen sollen. Dieser Mehrfachgebrauch beschränkt sich üblicherweise auf einen einzigen Tag.

Das Brusthaubenset in einer Ausführungsform weist mindestens einen Teil auf, welcher nicht autoklavierbar ist. Es genügt vollständig, wenn dies der Ventilkörper ist. Wird der Ventilkörper autoklaviert, so verzieht er sich und die Funktionalität des gesamten Brusthaubensets ist wegen mangelnder Dichtheit und somit ansteigendem Totvolumen nicht mehr gewährleistet.

Es können auch mehrere Teile aus nicht-antoklavierbarem Material gefertigt sein, wie beispielsweise der Ventilsitz, das Brusthaubenanschlussteil und der Brusthaubentrichter. Selbstverständlich können weitere Zubehörteile wie die Saugleitung aus einem nicht autoklavierbaren Material gefertigt sein.

### Die Lösung ergibt sich aus folgender Beobachtung;

Üblicherweise können Säuglings- oder Wöchnerinnenstationen der Spitäler die Brusthaubensets selber reinigen, so dass die Mütter jeweils ihr eigenes bisher benutztes Brusthaubenset wieder verwenden können. Des weiteren können die Mütter bzw. das Pflegepersonal dadurch kontrollieren, wie oft das Brusthaubenset schon verwendet worden ist, und es kann nach entsprechend kurzer Zeit entsorgt werden. Autoklavierbare Brusthaubenset werden üblicherweise in einer anderen Abteilung gereinigt, so dass sie die Säuglings- oder Wöchnerinnenstationen verlassen müssen, oft erst am nächsten Tag wieder auf die Station gelangen und die oben erwähnten Kontrollmöglichkeiten somit nicht mehr gegeben sind. Wird nun verhindert, dass die Brusthaubensets autoklaviert werden, ist der gewünschte kontrolliert beschränkte Mehrfachgebrauch möglich.

Durch die Forderung, dass der Ventilkörper nicht mehr autoklavierbar sein soll, wird die Materialwahl jedoch stark eingeschränkt. Als Material geeignet ist zwar ein elastomeres Polymer (TPE). Dieses Material stellt jedoch hohe Anforderungen an die Form des Ventilkörpers, damit er sich beim Reinigen nicht auch vergehen kann. Die Ventile gemäss den Patentansprüchen 1 bis 15 erfüllen diese Anforderungen.

Weitere vorteilhafte Ausführungsformen gehen aus den abhängigen Patentansprüchen hervor.

### Kurze Beschreibung der Zeichnungen

Im folgenden wird der Erfindungsgegenstand anhand von bevorzugten Ausführungsbeispielen, welche in der beiliegenden Zeichnung dargestellt sind, erläutert. Es zeigten:
- Figur 1: eine perspektivische Ansicht des Brusthaubensets inklusive Sammelbehälter, Saugleitung und Verschlussdeckel;
- Figur 2: eine Explosionsdarstellung des Brusthaubensets gemäss Figur 1;
- Figur 3: eine perspektivische Ansicht eines Brusthaubenanschlussteils;
- Figur 4a: eine perspektivische Ansicht eines Ventilsitzes;
- Figur 4b: eine weitere perspektivische Ansicht des Ventilsitzes gemäss Figur 4a;
- Figur 4c: eine Seitenansicht des Ventilsitzes gemäss Figur 4a;
- Figur 4d: eine Ansicht des Ventilsitzes gemäss Figur 4a von oben;
- Figur 4e: einen vergrösserten Querschnitt durch einen Teil des Ventilsitzes gemäss Figur 4a;
- Figur 5a: eine Seitenansicht eines Ventilkörpers;
- Figur 5b: eine Ansicht des Ventilkörpers gemäss Figur 5a von oben;
- Figur 5c: einen Querschnitt durch den Ventilkörper entlang A-A gemäss Figur 5b;
- Figur 5d: eine Ansicht des Ventilkörpers gemäss Figur 5a von unten;
- Figur 5e: eine perspektivische Darstellung des Ventilkörpers gemäss Figur 5a;
- Figur 5f: eine weitere perspektivische Darstellung des Ventilkörpers gemäss Figur 5a;
- Figur 5g: einen vergrösserten Ausschnitt P gemäss Figur 5b;
- Figur 5h: einen vergrösserten Ausschnitt Q gemäss Figur 5d;
- Figur 5i: einen vergrösserten Ausschnitt X gemäss Figur 5c;
- Figur 5k: einen vergrösserten Ausschnitt Y gemäss Figur 5c;
- Figur 6a: eine Seitenansicht eines Ventilkörpers gemäss einer zweiten Ausführungsform;
- Figur 6b: einen Querschnitt durch den Ventilkörper gemäss Figur 6a und
- Figur 6c: einen vergrösserten Ausschnitt gemäss Figur 6b.

### Wege zur Ausführung der Erfindung

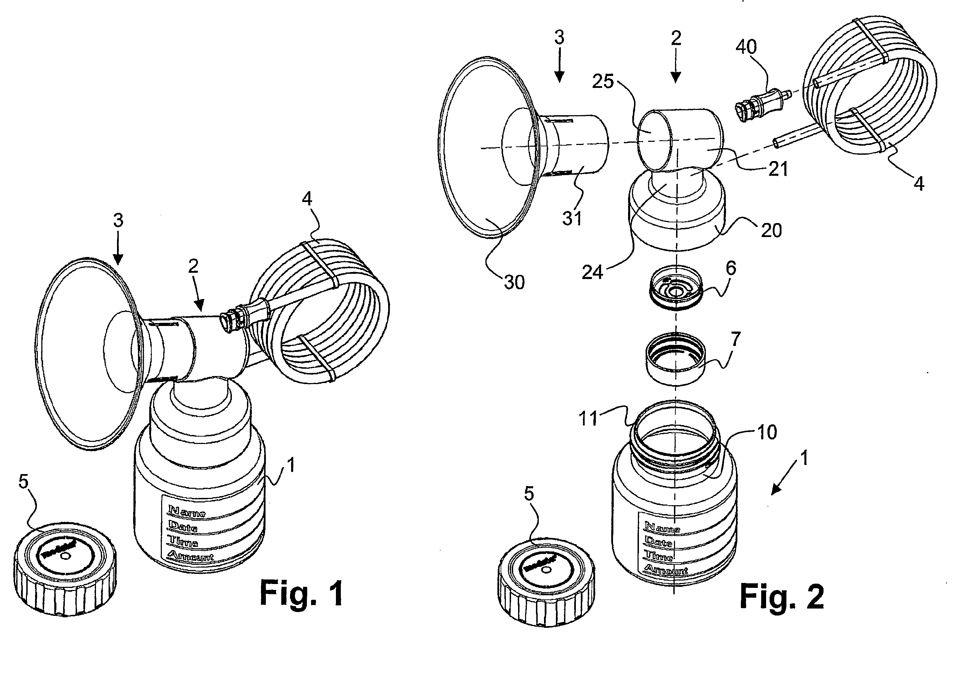

In Figur 1 ist ein erfindungsgemässes Brusthaubenset dargestellt. Auf einen Milchsammelbehälter 1 ist ein Brusthaubenanschlussteil 2 aufgeschraubt. Am Brusthaubenanschlussteil 2 ist einerseits eine Brusthaube 3 mit einem Brusthaubentrichter 30 und andererseits ist eine Saugleitung 4 befestigt. Anstelle des Brusthaubenanschlussteils 2, z.B. wenn der Sammelbehälter 1 gefüllt ist, lässt sich ein Verschlussdeckel 5 auf den Milchsammelbehälter 1 aufschrauben.

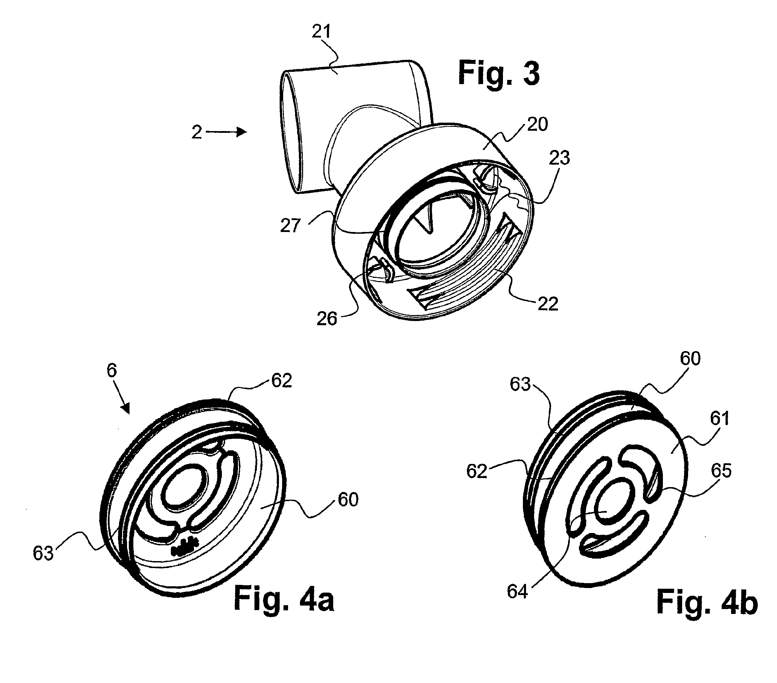

In Figur 2 ist ersichtlich, wie die einzelnen oben erwähnten Teile miteinander verbindbar sind. Der Sammelbehälter 1 weist einen Hals 10 mit einem Aussengewinde 11 auf, auf welches ein Gewindeaufsatz 20 mit einem Innengewinde 22 (Figur 3) des Brusthaubenanschlussteil 2 aufgeschraubt werden kann. An diesen Gewindeaufsatz 20 ist über einen kurzen Hals 24 ein Anschlussstutzen 21 angeformt. Der Anschlussstutzen 21 weist eine Aufnahmeöffnung 25 auf, in welche ein Kopplungsstutzen 31 der Brusthaube 3 eingesteckt werden kann. Am gegenüberliegenden Ende des Anschlussstutzens 21 ist eine nicht sichtbare Steckeraufnahme für den direkten Anschluss an die Saugleitung 4 vorhanden. Mit dem Kopplungsstück 40 kann der Schlauch 4 an eine externe Brustpumpe angeschlossen werden.

Die Brusthaube 3 kann auch einstückig am Brusthaubenanschlussteil 2 angeformt sein. Des weiteren kann der Brusthaubenanschlussteil 2 anstelle des Saugleitungssteckers eine Aufnahme für einen manuellen oder elektrischen Motor aufweisen.

Wie ebenfalls in Figur 2 dargestellt, ist zwischen Brusthaubenanschlussteil 2 und Sammelbehälter 1 ein Ventil mit einem Ventilsitz 6 mit einem daran befestigten Ventilkörper 7 vorhanden. Vorzugsweise ist dieses Ventil im Brusthaubenanschlussteil 2 angeordnet. Der Ventilsitz 6 kann einstückig am Brusthaubenanschlussteil 2 angeformt sein oder er kann auf eine entsprechende Aufnahme 23 aufsteckbar sein. Diese entsprechende Ventilaufnahme 23 in Form eines nach innen ragenden Halses ist in Figur 3 sichtbar. Die Bezugsziffer 26 bezeichnet obere Anschläge für den Milchsammelbehälter bzw. die Milchflasche.

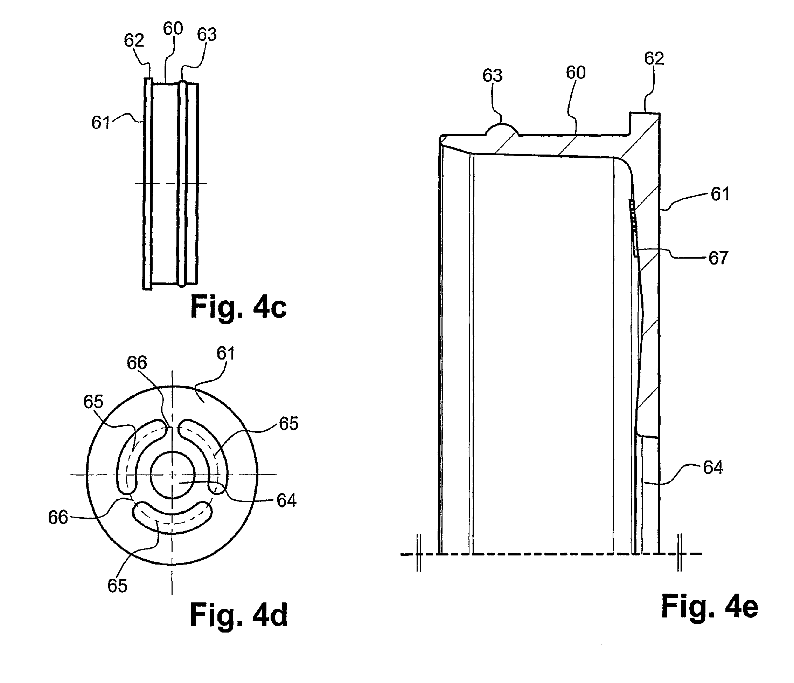

In den Figuren 4a bis 4e ist der Ventilsitz 6 dargestellt. Er weist eine kreisförmige, im wesentlichen oder absolut plane Deckfläche 61 und einen daran angeformten umlaufenden Kragen 60 auf. In der Deckfläche 61 ist eine zentrale Öffnung 64 und periphere Öffnungen 65 vorhanden. Die peripheren Öffnungen 65 bilden vorzugsweise einen gemeinsamen Kreis, deren Mittelpunkt mit dem Mittelpunkt der kreisförmigen Deckfläche 61 zusammenfällt. Vorzugsweise sind genau drei periphere Öffnungen 65 vorhanden, wobei der gemeinsame Kreis durch Stege 66 unterbrochen ist. Wie in den Figuren 4c und 4e am besten erkennbar ist, steht die Deckfläche 61 dem Kragen 60 in einem vorstehenden umlaufenden Rand 62 vor. Ferner weist der Kragen 60 im unteren, der Deckfläche 61 abgewandten Bereich ein umlaufender Wulst 63 auf. Das Teil lässt sich auch beschriften. Ein entsprechendes Beispiel ist in den Figuren mit der Bezugsziffer 67 versehen.

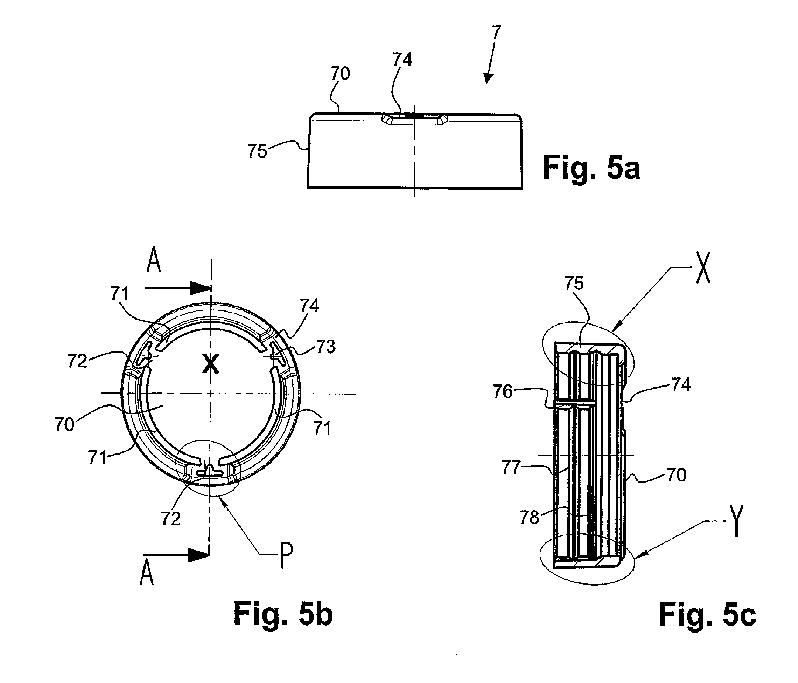

In den Figuren 5a bis 5k ist der Ventilkörper 7 dargestellt. Dieser Ventilkörper 7 lässt sich über den oben beschriebenen Ventilsitz 6 stülpen. Das Ventil wird im Brusthaubenanschlussteil 2 mit dem Wulst 27 bzw. vorzugsweise mit der ersten Nut 77 oder der zweiten Nut 78 gehalten.

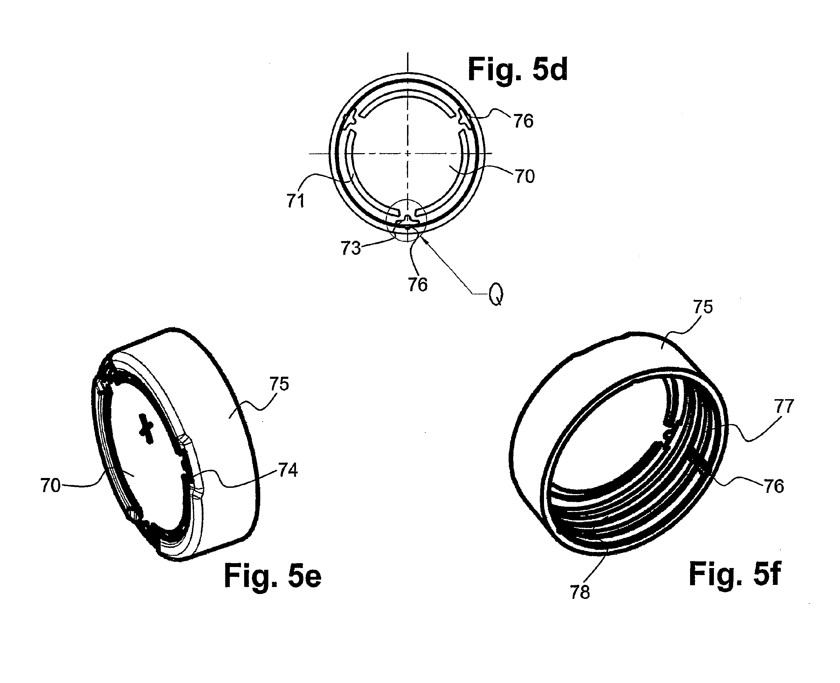

Der Ventilkörper 7 weist im wesentlichen dieselbe Grundform auf wie der Ventilsitz 6, d.h. er besitzt eine kreisförmige, im wesentlichen plane Membran 70, welche von einem umlaufenden zylinderförmigen Mantel 75 umgeben ist. Die Membran 70 kann auf ihrer dem Ventilsitz 6 abgewandten Aussenseite jedoch auch Noppen aufweisen. Die Membran 70 weist Bereiche 74 auf, welche geschwächt ausgebildet sind. Diese Schwächung kommt einerseits davon, dass diese Bereiche 74 eine geringere Materialdicke aufweisen, wie dies in Figur 5a ersichtlich ist Andererseits sind kompakte Öffnungen 73 vorhanden. Die verdünnten Bereiche oder Vertiefungen 74 weisen vorzugsweise einen stetig anwachsenden Übergang zur restlichen Membran auf. In Figur 5a ist ersichtlich, dass die Randbereiche dieser Bereiche 74 rampenförmig ausgebildet sind. Dies ist auch in Figur 5e erkennbar.

Wie insbesondere in Figur 5b dargestellt, ist die Membran 70 bis auf wenige Öffnungen 71, 73 im wesentlichen als geschlossene, mit ihrem Mantel 75 verbundenen Fläche ausgebildet und in einer Dreipunktaufhängung befestigt. Insbesondere sind Öffnungen 71, 73 nur im peripheren, jedoch nicht im mittleren Bereich vorhanden.

Die dargestellte Membran 70 weist zwei Typen von Öffnungen 71, 73 auf: schmale, langgezogene Öffnungen 71 und kleine, kompakte Öffnungen 73.

Die langgezogenen Öffnungen 71 sind im peripheren Bereich entlang des Umfangs der Membran 70 angeordnet. Im dargestellten Beispiel sind drei gleich lange und gleich grosse Öffnungen 71 vorhanden, wobei jede Öffnung 71 sich über einen Winkel von weniger als 120° erstreckt. Es kann jedoch auch eine andere Anzahl Öffnungen vorhanden sein. Vorzugsweise sind die Öffnungen 71 jedoch rotationssymmetrisch im peripheren Bereich der Membran angeordnet.

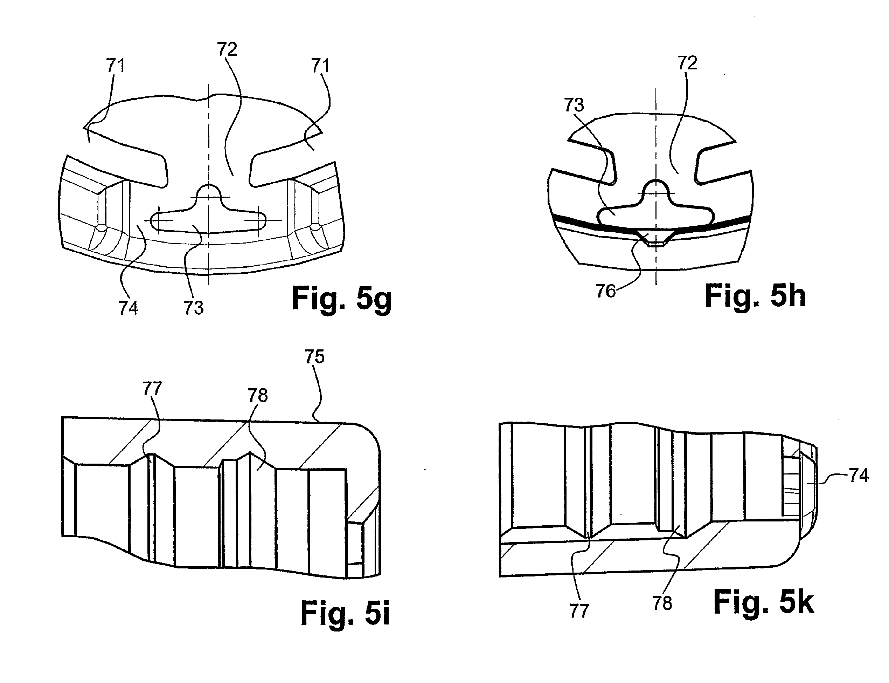

Sie bilden einen gemeinsamen Kreis, dessen Mittelpunkt vorzugsweise mit dem Mittelpunkt der Membran 70 oder Deckfläche zusammenfällt. Dieser Kreis ist von Stegen 72 durchbrochen. Im Bereich benachbart zu diesen Stegen 72 und an die peripheren Öffnungen 71 angrenzend sind die kleineren Öffnungen 73 angeordnet. Vorzugsweise befindet sich zwischen jeder langgezogenen Öffnung 71 eine kleine kompakte Öffnung 73. In diesem Beispiel sind somit ebenfalls drei dieser kompakten Öffnungen 73 vorhanden. Diese Öffnungen 73 sind T-förmig gestaltet mit einem Fuss und einem quer darüber verlaufenden Balken. Der Fuss ist dabei radial zu den Stegen und zum Mittelpunkt der Membran 70 hin gerichtet und der Balken ist nach aussen zum Umkreis hin gewandt. Vorzugsweise endet der Fuss an der Aussenseite des durch die peripheren Öffnungen 71 gebildeten Kreises. Die Balken der einzelnen Öffnungen 73 liegen vorzugsweise auf einem Kreis, dessen Mittelpunkt mit dem Mittelpunkt der Membran zusammenfällt. Die Balken sind vorzugsweise entsprechend diesem Kreis gebogen ausgebildet, wie dies in Figur 5g erkennbar ist.

Die T-förmigen Öffnungen 73 befinden sich vollständig innerhalb der geschwächten Bereiche oder Vertiefungen 74. Die Vertiefungen 74 sind jedoch vorzugsweise, mindestens im Bereich der Balken der Öffnungen 73, etwas breiter ausgebildet als die Öffnungen 73 selber.

Der Mantel 75 des Ventilkörpers 7 ist gemäss den Figuren 5c, 5i und 5k auf seiner Aussenseite vorzugsweise plan ausgebildet. Die Innenseite des Ventilkörpers 7 verfügt jedoch über mindestens eine, vorzugsweise zwei umlaufende Nuten 77, 78. Des weiteren ist mindestens eine sich quer zu den umlaufenden Nuten 77, 78 und somit parallel zu einer Zylindermittelachse erstreckende Kerbe 76 vorhanden, wie dies in Figur 5f dargestellt ist. Diese beginnt vorzugsweise bei der der Membran näher liegenden Nut 78 und endet an der äusseren Kante des Mantels 75. Im vorliegenden Beispiel sind drei Kerben 76 vorhanden, welche sich jeweils im Bereich der T-förmigen Öffnungen 73, vorzugsweise in der durch den Fuss definierten Linie befinden. Dies ist in den Figuren 5d und 5h erkennbar. Die Nuten 77, 78 und Kerben 76 tragen dazu bei, dass ein Verziehen des Mantels bei Temperaturänderungen, insbesondere beim Sterilisieren vermieden wird.

Wenn der Ventilkörper 7 auf dem Ventilsitz 6 angeordnet ist, wie dies sich aus der Zusammenschau der Figuren 4d und 5b ergibt, so sind die zentrale Öffnung 64 und die peripheren Öffnungen 65 des Ventilsitzes 6 von der kreisförmigen Membranklappe, welche sich innerhalb der langgezogenen Öffnungen 71 befindet, überdeckt. Die langgezogenen Öffnungen 71 und die kompakten Öffnungen 73 hingegen liegen auf dem äusseren Randbereich des Ventilsitzes 6 auf. Dadurch ist das Ventil bei Normaldruck geschlossen. Wird nun der Druck auf die Membranklappe erhöht, so wölbt sich diese gleichmässig zum Innenraum des Sammelbehälters 1 hin und geben die Öffnungen 64, 65 des Ventilsitzes frei. Dadurch entfernen sich auch die Öffnungen 71, 73 des Ventilkörpers 7 von ihrer Auflagefläche und es werden Durchgänge von einer Seite des Ventils auf die andere Seite gebildet. Lässt der Druck auf die Membraninnenseite nach, so senkt sich diese wieder auf den Ventilsitz ab und verschliesst das Rückschlagventil.

Die oben beschriebenen Einzelteile des Brusthaubensets sind vorzugsweise aus einem sterilisierbaren und autoklavierbaren Material, beispielsweise aus Polypropylen (PP) hergestellt. Mindestens ein Teil, vorzugsweise der Ventilkörper ist jedoch aus einem nicht-autoklavierbaren Material, beispielsweise einem thermoplastischen Elastomer (TPE) gefertigt. Vorzugsweise ist nur der Ventilkörper aus einem derartigen Material gefertigt.

Obwohl das erfindungsgemässe Ventil in seiner Verwendung in einem Brusthaubenset beschrieben wurde, lässt es sich auch in anderen Bereichen einsetzen, beispielsweise in Drainagebeuteln oder -behältern zur Absaugung von Körperflüssigkeiten, in Vakuumschläuchen oder anderen medizinischen oder sonstigen Vorrichtungen.

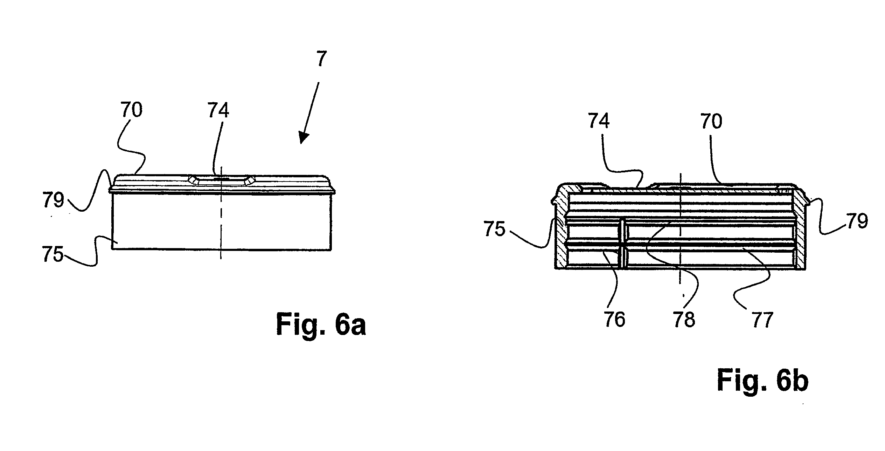

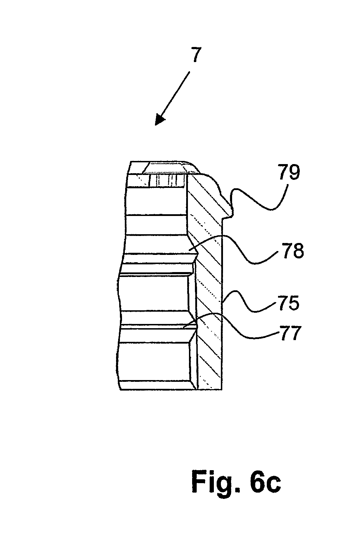

In den Figuren 6a bis 6c ist eine zweite bevorzugte Ausführungsform des Ventilkörpers 7 dargestellt. Er lässt sich mit dem oben beschriebenen Ventilsitz 6 verwenden. Bis auf einen äusseren Wulst 79 ist er gleich gestaltet wie das oben beschriebene erste Ausführungsbeispiel des Ventilkörpers 7. Vorzugsweise erstreckt sich dieser äussere Wulst 79 um den ganzen Umfang des Mantels 75. Er kann jedoch auch abschnittsweise unterbrochen sein. Ebenfalls vorzugsweise ist er im der Membran 70 benachbarten Randbereich des Mantels 75 angeordnet. Dank diesem Wulst 79 lässt sich der Ventilkörper 7 von Hand besser vom Ventilsitz 6 lösen, da der Wulst 79 die Griffigkeit der Oberfläche erhöht. Anstelle des oder zusätzlich zum Wulst 79 lässt sich auch eine Nut verwenden. Ebenso kann die Manteloberfläche mit Noppen, Vertiefungen oder axial verlaufenden Rippen versehen sein.

Das erfindungsgemässe Ventil ist kostengünstig und einfach herstellbar, zuverlässig in seiner Anwendung und verformt sich auch bei grösseren Temperaturschwankungen kaum.

### Bezugszeichenliste

- 1: Milchsammelbehälter
- 10: Hals
- 11: Gewinde
- 2: Brusthaubenanschlussteil
- 20: Gewindeaufsatz
- 21: Anschlussstutzen
- 22: Innengewinde
- 23: Ventilaufnahme
- 24: Hals
- 25: Aufnahmeöffnung
- 26: Anschlag
- 27: Wulst
- 3: Brusthaube
- 30: Brusthaubentrichter
- 31: Kopplungsstutzen
- 4: Saugleitung
- 40: Kopplungsstück
- 5: Verschlussdeckel
- 6: Ventilsitz
- 60: Kragen
- 61: Deckfläche
- 62: Rand
- 63: Wulst
- 64: Zentrale Öffnung
- 65: Periphere Öffnung
- 66: Steg
- 67: Beschriftung
- 7: Ventilkörper
- 70: Membran
- 71: Langgezogene Öffnung
- 72: Steg
- 73: Kompakte Öffnung
- 74: Vertiefung
- 75: Mantel
- 76: Kerbe
- 77: Erste Nut
- 78: Zweite Nut
- 79: Äusserer Wulst

## Patentansprüche

1. Ventil mit einem Ventilsitz (6) und einem Ventilkörper (7) mit einer kreisförmigen Membran (70), welcher über dem Ventilsitz (6) angeordnet ist und bei Auflage auf dem Ventilsitz (6) diesen dichtend verschliesst, wobei der Ventilsitz (6) und der Ventilkörper (7) Öffnungen (64, 65, 71, 73) aufweisen, welche versetzt zueinander angeordnet sind und bei Anhebung der Membran (70) des Ventilkörpers (7) einen freien Durchgang bilden, wobei die Membran (70) des Ventilkörpers (7) langgezogene Öffnungen (71) aufweist, welche gleichmässig verteilt entlang eines Kreises in der Peripherie der Membran (70) angeordnet sind und wobei die langgezogenen Öffnungen (71) durch Stege (72) voneinander getrennt sind, wobei die Membran (70) im Bereich (74) benachbart zu diesen Stegen (72) geschwächt ausgebildet ist, wobei benachbart zu den Stegen (72) kompakte Öffnungen (73) vorhanden sind, **dadurch gekennzeichnet, dass** die kompakten Öffnungen (73) T-förmig ausgebildet sind.

2. Ventil nach Anspruch 1, wobei der Kreis einen Mittelpunkt aufweist, welcher mit dem Mittelpunkt der kreisförmigen Membran (70) zusammen fällt.

3. Ventil nach einem der Ansprüche 1 oder 2, wobei die langgezogenen Öffnungen (71) einen gemeinsamen Kreisring bilden, dessen Breite ein Vielfaches kleiner als der kleinere Radius des Kreisrings ist und welcher mit den Stegen (72) versehen ist

4. Ventil nach einem der Ansprüche 1 bis 3, wobei genau drei langgezogene Öffnungen (71) und genau drei Stege (72) vorhanden sind.

5. Ventil nach einem der Ansprüche 1 bis 4, wobei die kompakten Öffnungen (73) im geschwächten Bereich (74) der Membran (70) angeordnet sind.

6. Ventil nach einem der Ansprüche 1 bis 5, wobei die T-förmigen Öffnungen (73) je einen Fuss und einen quer darüber verlaufenden Balken aufweisen und wobei der Fuss zu den Stegen (72) und radial zu einem Kreismittelpunkt der Membran (70) hin gerichtet ist.

7. Ventil nach einem der Ansprüche 1 bis 6, wobei der Ventilkörper (7) einen zylinderförmigen Mantel (75) aufweist, welcher die Membran (70) umgibt.

8. Ventil nach Anspruch 7, wobei die Membran (70) bis auf die langgezogenen und kompakten Öffnungen (71, 73) und geschwächten Bereichen (74) als plane, geschlossene Scheibe ausgebildet ist, welche umlaufend mit dem zylinderförmigen Mantel (75) verbunden ist.

9. Ventil nach einem der Ansprüche 7 oder 8, wobei der Mantel (75) mindestens eine Kerbe (76) aufweist, welche parallel zu einer Zylindermittelachse des zylinderförmigen Mantels (75) verläuft.

10. Ventil nach einem der Ansprüche 7 bis 9, wobei der zylinderförmige Mantel (75) eine Innenseite aufweist, welche mit mindestens einer mindestens teilweise umlaufenden Nut (77, 78) versehen ist.

11. Ventil nach einem der Ansprüche 7 bis 10, wobei der zylinderförmige Mantel (75) mit einem mindestens teilweise umlaufenden Wulst (79) versehen ist.

12. Ventil nach einem der Ansprüche 1 bis 11, wobei der Ventilsitz (6) eine plane Fläche (61) mit einer zentralen Öffnung (64) und diese zentrale Öffnung (64) umlaufende Öffnungen (65) aufweist, wobei die peripheren Öffnungen (65) durch Stege (66) unterbrochen sind.

13. Ventil nach einem der Ansprüche 1 bis 12, wobei mindestens ein Teil (7) des Ventils aus einem nicht-autoklavierbaren Material gefertigt ist.

14. Ventil nach Anspruch 13, wobei der Ventilkörper (7) aus einem nicht-autoklavierbaren Material gefertigt ist.

15. Ventil nach Anspruch 14, wobei der Ventilkörper (7) aus einem thermoplastischen Elastomer (TPE) gefertigt ist.

16. Brusthaubenset zum Abpumpen von menschlicher Muttermilch, wobei das Brusthaubenset eine Brusthaube (3), ein Brusthaubenanschlussteil (2) mit einem Gewindeaufsatz (20) zur Verbindung mit einem Milchsammelbehälter (1) und ein Ventil (6, 7) zur Begrenzung eines Totvolumens beim Abpumpen der Muttermilch aufweist, **dadurch gekennzeichnet, dass** das Ventil (6, 7) ein Ventil gemäss einem der Ansprüche 1 bis 15 ist.

17. Brusthaubenset nach Anspruch 16, wobei der Ventilsitz (6) des Ventils auf dem Brusthaubenanschlussteil (2) aufsteckbar ist oder einstückig an diesem angeformt ist.

18. Brusthaubenset nach den Ansprüchen 16 oder 17, wobei der Brusthaubenanschlussteil (2), die Brusthaube (3) und der Ventilsitz (6) aus einem autoklavierbaren Material und der Ventilkörper (7) aus einem nicht-autoklavierbaren Material gefertigt sind.

19. Brusthaubenset nach Anspruch 18, wobei das autoklavierbare Material Polypropylen (PP) und das nicht-autoklavierbare Material ein thermoplastisches Elastomer (TPE) ist.

## Claims

1. A valve having a valve seat (6) and a valve body (7) with a circular diaphragm (70), the valve body (7) being arranged over the valve seat (6) and closing the latter sealingly when it bears on said valve seat (6), and the valve seat (6) and valve body (7) having openings (64, 65, 71, 73) which are offset relative to one another and which form a free passage when the diaphragm (70) of the valve body (7) lifts, wherein the diaphragm (70) of the valve body (7) has elongate openings (71) which are uniformly distributed along a circle in the periphery of the diaphragm (70), and wherein the elongate openings (71) are separated from one another by webs (72), the diaphragm (70) being designed to be weaker in the area (74) adjacent to these webs (72), **characterized in that** the compact openings (73) have a T-shaped configuration.

2. The valve as claimed in claim 1, in which the circle has a center point that coincides with the center point of the circular diaphragm (70).

3. The valve as claimed in either of claims 1 and 2, in which the elongate openings (71) form a common circular ring whose width is a multiple smaller than the smaller radius of the circular ring and which is provided with webs (72).

4. The valve as claimed in one of claims 1 through 3, in which exactly three elongate openings (71) and exactly three webs (72) are present.

5. The valve as claimed in one of claims 1 through 4, in which the compact openings (73) are arranged in the weakened area (74) of the diaphragm (70).

6. The valve as claimed in claim 6, wherein the T-shaped openings (73) each have a foot and a bar extending transversely over the latter, and in which the foot is oriented toward the webs (72) and radially toward a center point of the circle of the diaphragm (70).

7. The valve as claimed in one of claims 1 through 6, in which the valve body (7) has a cylindrical jacket (75) that surrounds the diaphragm (70).

8. The valve as claimed in claim 7, wherein the diaphragm (70), except for the elongate openings and compact openings (71, 73) and weakened areas (74), is designed as a plane, closed disk, which is connected circumferentially to the cylindrical jacket (75).

9. The valve as claimed in either of claims 7 and 8, in which the jacket (75) has at least one notch (76) extending parallel to a center axis of the cylindrical jacket (75).

10. The valve as claimed in one of claims 7 through 9, in which the cylindrical jacket (75) has an inner face provided with at least one groove (77, 78) extending at least partially about the circumference.

11. The valve as claimed in one of claims 7 through 10, in which the cylindrical jacket (75) is provided with a bead (79) extending at least partially about the circumference.

12. The valve as claimed in one of claims 1 through 11, in which the valve seat (6) has a plane surface (61) with a central opening (64) and with openings (65) extending around this central opening (64), the peripheral openings (65) being interrupted by webs (66).

13. The valve as claimed in one of claims 1 through 12, in which at least one part (7) of the valve is made from a non-autoclavable material.

14. The valve as claimed in claim 13, in which the valve body (7) is made from a non-autoclavable material.

15. The valve as claimed in claim 14, in which the valve body (7) is made from a thermoplastic elastomer (TPE).

16. A breast shield set for pumping off human breast milk, the breast shield set comprising a breast shield (3), a breast shield connector (2) with a threaded attachment (20) for connection to a milk collection vessel (1), and a valve (6, 7) for limiting a dead volume during pumping off of breast milk, wherein the valve (6, 7) is a valve as claimed in one of claims 1 through 15.

17. The breast shield set as claimed in claim 16, in which the valve seat (6) of the valve can be fitted onto the breast shield connector (2) or is formed integrally on the latter.

18. The breast shield set as claimed in claim 16 or 17, in which the breast shield connector (2), the breast shield (3) and the valve seat (6) are made from an autoclavable material and the valve body (7) is made from a non-autoclavable material.

19. The breast shield set as claimed in claim 18, in which the autoclavable material is polypropylene (PP) and the non-autoclavable material is a thermoplastic elastomer (TPE).

## Revendications

1. Soupape comprenant un siège de soupape (6) et un corps de soupape (7) avec une membrane de forme circulaire (70), lequel est disposé par-dessus le siège de soupape (6) et le ferme hermétiquement lors de l'application sur le siège de soupape (6), le siège de soupape (6) et le corps de soupape (7) présentant des ouvertures (64, 65, 71, 73) qui sont disposées de manière décalée les unes par rapport aux autres et qui, lors du soulèvement de la membrane (70) du corps de soupape (7), forment un passage libre, la membrane (70) du corps de soupape (7) présentant des ouvertures allongées (71), lesquelles sont disposées de manière répartie uniformément le long d'un cercle dans la périphérie de la membrane (70), les ouvertures allongées (71) étant séparées les unes des autres par des nervures (72), la membrane (70) étant réalisée sous forme affaiblie dans la région (74) adjacente à ces nervures (72), des ouvertures compactes (73) étant prévues à côté des nervures (72), **caractérisée en ce que** les ouvertures compactes (73) sont réalisées en forme de T.

2. Soupape selon la revendication 1, dans laquelle le cercle présente un centre qui coïncide avec le centre de la membrane de forme circulaire (70).

3. Soupape selon l'une quelconque des revendications 1 et 2, dans laquelle les ouvertures allongées (71) forment une bague circulaire commune dont la largeur est inférieure d'un multiple au plus petit rayon de la bague circulaire et qui est pourvue des nervures (72).

4. Soupape selon l'une quelconque des revendications 1 à 3, dans laquelle exactement trois ouvertures allongées (71) et exactement trois nervures (72) sont prévues.

5. Soupape selon l'une quelconque des revendications 1 à 4, dans laquelle les ouvertures compactes (73) sont disposées dans la région affaiblie (74) de la membrane (70).

6. Soupape selon l'une quelconque des revendications 1 à 5, dans laquelle les ouvertures en forme de T (73) présentent chacune une base et une poutre s'étendant transversalement au-dessus de celle-ci et dans laquelle la base est orientée vers les nervures (72) et radialement vers un centre du cercle de la membrane (70).

7. Soupape selon l'une quelconque des revendications 1 à 6, dans laquelle le corps de soupape (7) présente une enveloppe cylindrique (75) qui entoure la membrane (70).

8. Soupape selon la revendication 7, dans laquelle la membrane (70) est réalisée, à l'exception des ouvertures allongées et compactes (71, 73) et des régions affaiblies (74), sous forme de disque plan fermé qui est connecté sur sa périphérie à l'enveloppe cylindrique (75).

9. Soupape selon l'une quelconque des revendications 7 et 8, dans laquelle l'enveloppe (75) présente au moins une entaille (76) qui s'étend parallèlement à un axe médian cylindrique de l'enveloppe cylindrique (75).

10. Soupape selon l'une quelconque des revendications 7 à 9, dans laquelle l'enveloppe cylindrique (75) présente un côté interne qui est pourvu d'au moins une rainure au moins partiellement périphérique (77, 78).

11. Soupape selon l'une quelconque des revendications 7 à 10, dans laquelle l'enveloppe cylindrique (75) est pourvue d'un bourrelet au moins partiellement périphérique (79).

12. Soupape selon l'une quelconque des revendications 1 à 11, dans laquelle le siège de soupape (6) présente une surface plane (61) avec une ouverture centrale (64) et des ouvertures (65) entourant cette ouverture centrale (64), les ouvertures périphériques (65) étant interrompues par des nervures (66).

13. Soupape selon l'une quelconque des revendications 1 à 12, dans laquelle au moins une partie (7) de la soupape est fabriquée en un matériau ne pouvant pas être traité en autoclave.

14. Soupape selon la revendication 13, dans laquelle le corps de soupape (7) est fabriqué en un matériau ne pouvant pas être traité en autoclave.

15. Soupape selon la revendication 14, dans laquelle le corps de soupape (7) est fabriqué en un élastomère thermoplastique (TPE).

16. Ensemble téterelle pour pomper du lait maternel humain, l'ensemble téterelle présentant une téterelle (3), une partie de raccordement à la téterelle (2) avec un embout fileté (20) pour la connexion à un récipient de collecte de lait (1) et une soupape (6, 7) pour limiter un volume mort lors du pompage du lait maternel, **caractérisé en ce que** la soupape (6, 7) est une soupape selon l'une quelconque des revendications 1 à 15.

17. Ensemble téterelle selon la revendication 16, dans lequel le siège de soupape (6) de la soupape peut être enfiché sur la partie de raccordement à la téterelle (2) ou est façonné d'une seule pièce sur celle-ci.

18. Ensemble téterelle selon la revendication 16 ou 17, dans lequel la partie de raccordement à la téterelle (2), la téterelle (3) et le siège de soupape (6) sont fabriqués en un matériau pouvant être traité en autoclave et le corps de soupape (7) est fabriqué en un matériau ne pouvant pas être traité en autoclave.

19. Ensemble téterelle selon la revendication 18, dans lequel le matériau pouvant être traité en autoclave est un polypropylène (PP) et le matériau ne pouvant pas être traité en autoclave est un élastomère thermoplastique (TPE).
